# EUROPEAN PATENT APPLICATION

(11) **EP 1 832 586 A1**
(43) Date of publication of application: **12.09.2007**
(21) Application number: 06110952.6
(22) Date of filing: 10.03.2006
(51) Int. Cl.: C07D 417/04, C07D 277/10, C07D 211/32, A61K 31/427, A61K 31/4418, A61P 1/16, A61P 1/18

(54) **Thiazole-piperidine derivatives in treatment of diseases of liver and the pancreas**

(71) Applicant: Oridis Biomed Forschungs- und Entwicklungs GmbH, 8010 Graz (AT)
(72) Inventor: Otte, Marcus, 8010 GRAZ (AT)

(57) **Abstract**

The present invention relates to the use of piperidinyl-thiazole derivatives of formula (1) 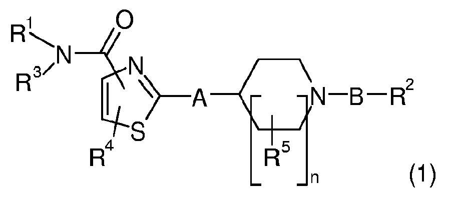 in the treatment and prevention of diseases of liver and the pancreas, wherein the substituents have the meanings explained in the description, and to particular compounds suitable for this use and to pharmaceutical compositions containing them.

## Description

### Technical Field

The present invention relates to the use of thiazole-piperidine compounds, such as piperidinyl-thiazole carboxylic acid and carboxamide derivatives in the treatment and prevention of diseases of liver and of the pancreas, e.g. liver- and pancreatic cancers.

### Background Art

The spectrum of liver disease varies from mild and reversible fatty liver to progressive chronic liver disease, which results in the development of the life threatening conditions of liver cancer, liver cirrhosis and liver failure.

Although liver cancer is relatively uncommon in the industrialized western world, it is among the leading causes of cancer worldwide. In contrast to many other types of cancer, the number of people who develop and die from liver cancer is increasing. On a global basis, primary liver cancer such as HCC belongs to the most common malignant tumors accounting for about 1 million deaths/ year (Bruix, J. et al., 2004, Cancer Cell (5): 215-219).

The principal risk factors for liver cancer, such as hepatocarcinoma (HCC) are viruses, alcohol consumption, food contamination with aflatoxin molds and metabolic disorders. The rates of alcoholism and chronic hepatitis B and C continue to increase. The outlook therefore is for a steady increase in liver cancer rates, underscoring the need for new therapies in this area.

Primary liver cancer is difficult to treat. Surgical removal of the cancer and liver transplantation is limited to small cancers and not a viable option for most patients since at diagnosis the cancer is often in an advanced stage. Chemotherapy is occasionally used for tumors not suitable for surgery but any benefit is usually short lived. Thus, survival rates for primary liver cancer are particularly low. Conventional therapy has generally not proven effective in the management of liver cancer.

For HCC for instance, there is no effective therapeutic option except resection and transplantation but these approaches are only applicable in early stages of HCC, limited by the access to donor livers, and associated with severe risks for the patient. In addition, these approaches are extremely expensive. These cancers respond very poorly to chemotherapeutics, most likely due to the normal liver function in detoxification and export of harmful compounds. Several other therapeutic options, such as chemoembolization, cryotherapy and ethanol injection are still in an experimental phase and the efficacy of these is not established. Thus until now no satisfactory therapies have been developed in order to be able to intervene in liver diseases.

The major causes of chronic liver disease are infections with hepatitis B or C virus, excessive consumption of alcohol and non-alcoholic fatty liver disease (NAFLD).

Hepatitis B virus (HBV) infection is a global public health issue. It is the leading cause of liver cirrhosis and HCC worldwide (Conjeevaram H.S. et al., 2003, Journal of Hepatology, 38: 90-103). Hepatitis C virus (HCV), a major cause of liver-related morbidity and mortality worldwide, represents one of the main public health problems (Alberti A. and Benvegnù L., Journal of Hepatology 2003, 38: 104-118). The HCV infection frequently causes chronic hepatitis, which is linked to the development of liver cirrhosis and HCC (Cyong J.C. et al., 2002, Am J Chin Med, 28: 351-360).

Alcoholic liver disease (ALD) is the commonest cause of cirrhosis in the Western world, currently one of the ten most common causes of death. In the United States, ALD affects at least 2 million people, or approximately 1 % of the population. The true incidence of ALD, especially in its milder forms, may be substantially greater because many patients are asymptomatic and may never seek medical attention. The spectrum of ALD ranges from fatty liver (steatosis), present in most, if not all heavy drinkers, through steatohepatitis, cholestasis (characterised by blocked bile excretion from the liver), fibrosis and ultimately cirrhosis (Stewart S.F. and Day C.P, 2003, Journal of Hepatology, 38: 2-13). Although fatty liver is reversible with abstention, it is a risk factor for progression to fibrosis and cirrhosis in patients who continue drinking, particularly when steatohepatitis is present.

Non-alcoholic fatty liver disease (NAFLD) refers to a wide spectrum of liver damage, ranging from simple steatosis to steatohepatitis, cholestasis, advanced fibrosis and cirrhosis. Steatohepatitis (non-alcoholic steatohepatitis) represents only a stage within the spectrum of NAFLD (Anguilo P., 2002, New Engl. J. Med., 346: 1221-1231). The pathological picture resembles that of alcohol-induced liver injury, but it occurs in patients who do not abuse alcohol. NAFLD should be differentiated from steatosis, with or without hepatitis, resulting from secondary causes, because these conditions have distinctly different pathogens and_outcomes. These secondary causes of fatty liver disease (steatosis) are nutritional (e.g. protein-calorie malnutrition, starvation, total parenteral nutrition, rapid weight loss, gastrointestinal surgery for obesity), drugs (e.g. glucocorticoids, synthetic estrogens, aspirin, calcium-channel blockers, tetracycline, valproic acid, cocaine, antiviral agents, fialuridine, interferon a, methotrexate, zidovudine), metabolic or genetic ( e.g. lipodostrophy, dysbetalipoproteinemia, Weber-Christian disease, galactosaemia, glycogen storage disorders, acute fatty liver of pregnancy) and other, such as diabetes mellitus, obesity or hyperlipidaemia (Anguilo P., 2002, New Engl. J. Med., 346: 1221-1231; MacSween R.N.M. et al., 2002, Pathology of the Liver. Fourth Edition. Churchill Livingstone, Elsevier Science).

Despite the prevalence of chronic liver diseases effective therapies for most disorders in this category are absent.

The severest of the non-viral chronic liver diseases, alcoholic steatohepatitis and non-alcoholic steatohepatitis (ASH and NASH) lead with high frequency to liver cirrhosis, liver failure and liver cancer (e.g. HCC). ASH and NASH cannot be distinguished by morphologic evaluation in the diagnostic pathology laboratory. Increased fatty disposition accompanied by fibrosis, inflammation and alterations in liver cell (hepatocyte) morphology, however, indicate these more serious conditions. Cellular changes in ASH and NASH include increased size (ballooning) and presence of intracellular aggregates (e.g. Mallory bodies), and this spectrum of liver cell pathology is considered to be diagnostic for these conditions. Overall, there is no proven specific treatment for ASH and NASH, having a definitive diagnosis via biopsy is not very likely to affect the management of the disease in a patient.

The pancreatitis representing one of the most frequent diseases of the pancreas can be an acute or chronic inflammation of the pancreas. Acute attacks often are characterized by severe abdominal pain that radiates from the upper stomach through to the back and can cause effects ranging from mild pancreas swelling to life-threatening organ failure. Chronic pancreatitis is a progressive condition that may involve a series of acute attacks, causing intermittent or constant pain as it permanently damages the pancreas. While the exact mechanisms of pancreatitis are not well understood, it is more frequent in men than in women and is known to be linked to and aggravated by alcoholism and gall bladder disease (gallstones that block the bile duct where it runs through the head of the pancreas and meets the pancreatic duct, just as it joins the duodenum). These two conditions are responsible for about 80% of acute pancreatitis attacks and figure prominently in chronic pancreatitis. Another 10% of the time the cause is idiopathic (unknown) and 10% of the time it is due to: pancreatic cancer, drugs such as valproic acid and estrogen, viral infections such as mumps, Epstein-Barr, and hepatitis A and B, hypertriglyceridemia, hyperparathyroidism, or hypercalcemia, cystic fibrosis or Reye's syndrome in children, surgery in the pancreas area (such as bile duct surgery) or trauma (http://www.labtestsonline.org/understanding/conditions/pancreatitis.html).

Most (95%) pancreatic cancers are adenocarcinomas that develop in the ducts and sometimes in the enzyme producing cells of the exocrine tissue. Endocrine tumors are usually less aggressive than exocrine tumors and are much rarer. They may be benign tumors (those that do not metastasize, such as insulinomas) or malignant (a group of cancers called islet cell cancers). They often are detected earlier than exocrine cancers because they tend to produce excessive amounts of the hormones insulin and glucagon.

According to the American Cancer Society, the pancreatic cancer is the fourth leading cause of cancer death in the United States, primarily because only about 10% of the cancers are still contained within the pancreas at the time of diagnosis.

It is already known that thiazole-piperidine compounds are specific to vascular endothelial growth factor (VEGF) or EP3 receptor, thus useful in treating of VEGF- mediated disorders (particularly endometrosis and acute macular degenerative disorder), and alteration of vascular tone, including myometrial and endometrial vascular tone, respectively (published in WO 2004/058750 and WO 2004/058751). These compounds, however, were not disclosed for use in treatment or prophylaxis of diseases of liver and/or pancreas.

### Summary of the Invention

The invention relates to the use of thiazol-piperidine derivatives, namely piperidinyl-thiazole carboxylic acid and carboxamide derivatives in the treatment of diseases of liver and pancreas.

Further it relates to particular compounds useful in the treatment of diseases of liver and pancreas and to pharmaceutical preparations containing them.

### Detailed Description

It has now unexpectedly been found that the piperidinyl-thiazole-derivatives disclosed in WO 2004/058750 and WO 2004/058751 are useful in the treatment and prevention of diseases of liver and the pancreas.

In its broadest aspect, the invention provides a compound of formula (1) 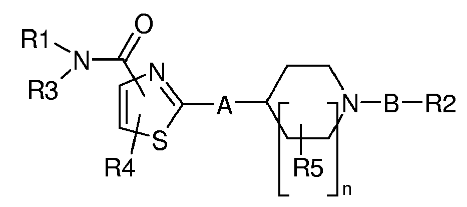 wherein
R¹ is optionally substituted alkyl, alkenyl, cycloalkyl, heterocyclyl, aryl, arylalkyl, heteroaryl, heteroarylalkyl, alkoxy, amino, alkylamino, dialkylamino, alkanoylamino, aroylamino, carboxyalkyl, alkoxycarbonyl, aryloxycarbonyl, or cyano;
R² is optionally substituted alkyl, alkenyl, alkinyl, cycloalkyl, heterocyclyl, aryl, arylalkyl, aryloxyalkyl, heteroaryl, heteroarylalkyl, or carboxyalkyl;
R³ is hydrogen or forms a ring together with R¹;
R⁴ is hydrogen or alkyl
R⁵ is alkyl and n is any number from zero to nine;
A is a single bond or alkylene;
B is a single bond, carbonyl, carbonyloxy, carbonylmercapto, carbonylamino wherein nitrogen is optionally further substituted by alkyl or is part of a ring, thiocarbonyl, thiocarbonyloxy, thiocarbonylmercapto, thiocarbonylamino, sulfonyl, or sulfonylamino;
and wherein the residue R¹NR³CO and R⁴ can be connected either to the carbon atom bound to sulfur or bound to nitrogen of the thiazole ring and the alkyl residues (R⁵)ₙ may be bound to any carbon atom of the piperidine ring;
for use in the treatment and prevention of diseases of liver and the pancreas.
In particular, the compounds according to the invention are useful for the treatment of hyperproliferative liver diseases, such as cancers, preferably hepatocarcinoma. Furthermore, the compounds according to the invention are useful for the treatment of hyperproliferative diseases of pancreas, such as cancers of pancreas, preferably adenocarcinoma of pancreas.

The general terms used hereinbefore and hereinafter preferably have within the context of this disclosure the following meanings, unless otherwise indicated:

The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Double bonds in principle can have E- or Z-configuration. The compounds of this invention may therefore exist as isomeric mixtures or single isomers. If not specified both isomeric forms are intended.

Any asymmetric carbon atoms may be present in the (R)-, (S)- or (R,S)-configuration, preferably in the (R)- or (S)-configuration. The compounds may thus be present as mixtures of isomers or as pure isomers, preferably as enantiomer-pure diastereomers.

The invention relates also to possible tautomers of the compounds of formula (I).

Alkyl has from 1 to 12, preferably from 1 to 10 carbon atoms, and is linear or branched. Alkyl is preferably lower alkyl, for example, C₁-C₇-alkyl, preferably C₁-C₄-alkyl.

Lower alkyl has 1 to 7 carbon atoms, preferably 1 to 4 carbon atoms, and is e.g. n-hexyl, n-pentyl, butyl, such as n-butyl, sec-butyl, isobutyl or tert-butyl, propyl, such as n-propyl or isopropyl, ethyl or methyl. Most preferably lower alkyl is methyl or ethyl.

Cycloalkyl has preferably 3 to 7 ring carbon atoms, and may be unsubstitued or substituted, e.g. by lower alkyl or lower alkoxy. C₃-C₇-cycloalkyl is, for example, cyclopheptyl, cyclohexyl, cyclopentyl, methylcyclopentyl or cyclopropyl.

Aryl stands for a mono- or bicyclic fused ring aromatic group with 5 to 10 carbon atoms, such as phenyl, 1-naphthyl or 2-naphthyl, or also a partially saturated bicyclic fused ring comprising a phenyl group, such as indanyl, dihydro- or tetrahydronaphthyl. Preferably, aryl is phenyl.

In optionally substituted aryl or phenyl, substituents are preferably lower alkyl, lower alkoxy, lower alkoxy-lower alkoxy, methylenedioxy, ethylenedioxy, halo-lower alkyl, lower alkoxy-lower alkyl, aminocarbonyl, lower alkoxycarbonyl, lower alkylsulfonyl, halo, or nitro.

Heteroaryl represents an aromatic group containing at least one heteroatom selected from nitrogen, oxygen and sulfur, and is mono- or bicyclic. Monocyclic heteroaryl includes 5 or 6 membered heteroaryl groups containing 1, 2, 3 or 4 heteroatoms selected from nitrogen, sulfur and oxygen. Bicyclic heteroaryl includes 9 or 10 membered fused-ring heteroaryl groups. Examples of heteroaryl include pyrrolyl, thienyl, furyl, pyrazolyl, imidazolyl, triazolyl, oxazolyl, isoxazolyl, oxadiazolyl, thiazolyl, isothiazolyl, thiadiazolyl, pyridyl, pyridazinyl, pyrimidinyl, pyrazinyl, and benzo fused derivatives of such monocyclic heteroaryl groups, such as indolyl, benzimidazolyl or benzofuryl, quinolinyl, isoquinolinyl, quinazolinyl, quinoxalinyl or purinyl, preferably pyrrolyl, thienyl, furyl, pyrazolyl, imidazolyl, triazolyl, oxazolyl, thiadiazolyl, pyridyl, pyrimidinyl or pyrazinyl.

In optionally substituted heteroaryl, substituents are preferably lower alkyl, halo-lower alkyl, lower alkoxy-lower alkyl, cycloalkyl, phenyl, thienyl, lower alkoxy, lower alkoxy-lower alkoxy, lower alkylthio, lower alkenylthio, amino optionally substituted by one or two substituents lower alkyl and one substituent lower alkylcarbonyl, aminocarbonyl, halo, or nitro.

If one structure of aryl or heteroaryl is shown by structure in a formula or designated by chemical name, corresponding tautomers are also included.

Alkenyl contains one or more, e.g. two or three, double bonds, and is preferably lower alkenyl, such as C₁-C₄-alkenyl, e.g. 1- or 2-butenyl, 1-propenyl, allyl or vinyl.

Alkinyl is preferably lower alkinyl, such as C₁-C₄-alkinyl, e.g. propargyl or acetylenyl.

In optionally substituted alkenyl or alkinyl, substituents are preferably lower alkyl, lower alkoxy, halo or di(lower alkyl)amino, and are connected with a saturated carbon atom of alkenyl or alkinyl or with an unsaturated carbon atom of alkenyl.

Heterocyclyl designates preferably a saturated, partially saturated or unsaturated, mono- or bicyclic ring containg 4-10 atoms comprising one, two or three heteroatoms selected from nitrogen, oxygen and sulfur, which may, unless otherwise specified, be carbon or nitrogen linked, wherein a ring nitrogen atom may optionally be substituted by a group selected from lower alkyl, amino-lower alkyl, aryl, heteroaryl, aryl-lower alkyl, aryl-lower alkenyl and acyl, and a ring carbon atom may be substituted by lower alkyl, amino-lower alkyl, aryl, aryl-lower alkyl, heteroaryl, lower alkoxy, hydroxy or oxo. Examples of heterocyclyl are pyrrolidinyl, imidazolidinyl, pyrazolidinyl, oxazolidinyl, isoxazolidinyl, thiazolidinyl, isothiazolidinyl, piperidinyl, morpholinyl, piperazinyl, dioxolanyl and tetrahydropyranyl.

A ring formed by substituent R³ together with R¹ includes the nitrogen atom, to which R³ and R¹ are bound, and is preferably a heterocyclic ring as defined hereinbefore comprising at least one nitrogen atom. Such a ring may, for example, be pyrrolidinyl, imidazolidinyl, pyrazolidinyl, oxazolidinyl, thiazolidinyl, piperidinyl, morpholinyl, or piperazinyl, optionally substituted as defined hereinbefore, and is preferably piperazinyl, further substituted at the nitrogen atom in position 4 of piperazine by optionally substituted phenyl, optionally substituted phenylalkyl, optionally substituted phenylalkenyl, optionally substituted pyridyl, or pyrimidinyl.

Acyl designates, for example, alkylcarbonyl, cyclohexylcarbonyl, arylcarbonyl, aryl-lower alkylcarbonyl, or heteroarylcarbonyl. Lower acyl is preferably C₁-C₄-alkylcarbonyl, in particular propionyl or acetyl.

Hydroxyalkyl is especially hydroxy-lower alkyl, preferably hydroxy-C₁-C₄-alkyl, e.g. hydroxymethyl, 2-hydroxyethyl or 2-hydroxy-2-propyl.

Haloalkyl is especially halo-lower alkyl, preferably halo-C₁-C₄-alkyl, e.g. fluoroalkyl, especially trifluoromethyl, pentafluoroethyl or 3,3,3-trifluoroethyl.

Lower alkoxy is especially C₁-C₄-alkoxy, e.g. methoxy, ethoxy, isopropyloxy, or tert-butyloxy.

Arylalkyl includes aryl and alkyl as defined hereinbefore, and is e.g. benzyl, 1-phenethyl or 2-phenethyl.

Heteroarylalkyl includes heteroaryl and alkyl as defined hereinbefore, and is e.g. 2-pyridyl-methyl, 3-pyridyl-methyl, 4-pyridyl-methyl, 1- or 2-pyrrolyl-methyl, 3-indolyl-methyl, or 1-imidazolyl-methyl.

In substituted amino, the substituents are preferably lower alkyl, cycloalkyl, cycloalkyl-lower alkyl, hydroxy-lower alkyl, lower alkoxy-lower alkyl, hydroxy-lower alkoxy-lower alkyl, lower alkoxy-lower alkoxy-lower alkyl, optionally substituted phenyl, optionally substituted phenyl-lower alkyl, optionally substituted heteroaryl, optionally substituted heteroaryl-lower alkyl or lower alkylcarbonyl, or the two substituents on nitrogen form together with the nitrogen heteroaryl or heterocyclyl. In particular, substituted amino is C₁-C₄-alkylamino, di-C₁-C₄-alkylamino, optionally substituted phenylamino or optionally substituted phenyl-C₁-C₄-alkylamino.

Salts are especially the pharmaceutically acceptable salts of compounds of formula (1).

The term "liver disease" refers to and comprises all kinds of disorders that preferably affect the anatomy, physiology, metabolic, and/or genetic activities of the liver, that preferably affect the generation of new liver cells, and/or the regeneration of the liver, as a whole or parts thereof preferably transiently, temporarily, chronically or permanently in a pathological way. Preferably are included hyperproliferative diseases of liver such as neoplastic liver diseases (liver tumors). The term "hyperproliferative disease of liver" includes any liver disease wherein the rate of cell proliferation defined e.g. by mitotic index, is higher than in normal healthy liver.

Within the meaning of the present invention the term "liver disease" preferably also encompasses liver cancer. The term "liver cancer" within the meaning of the invention includes carcinomas in the liver, preferably hepatocellular carcinoma (HCC), metastases in liver originated from any organ (e.g. colon, breast), cholangicarcinoma, in which epithelial cell components of the tissue are transformed resulting in a malignant tumor identified according to the standard diagnostic procedures as generally known to a person skilled in the art. Preferably HCC further comprises subtypes of the mentioned disorders, preferably including liver cancers characterized by intracellular proteinaceous inclusion bodies, HCCs characterized by hepatocyte steatosis, and fibrolamellar HCC. For example, precancerous lesions are preferably also included such as those characterized by increased hepatocyte cell size (the "large cell" change), and those characterized by decreased hepatocyte cell size (the "small cell" change) as well as macro regenerative (hyperplastic) nodules (Anthony, P. in MacSween et al, eds. Pathology of the Liver. 2001, Churchill Livingstone, Edinburgh). Liver disease is further understood to comprise hyperproliferative diseases of liver e.g. benign liver neoplasms such as liver cell adenoma and/or focular nodular hyperplasia (FNH).

Liver disease further comprises any liver disorders caused by trauma, intoxication, in particular by alcohol, drugs or food intoxication, radiation, infection, cholestasis, immune reactions. Furthermore are included inherited liver diseases and inherited metabolic liver diseases. Preferred examples of liver diseases include cirrhosis, alcoholic and nonalcoholic liver disease, chronic hepatitis, Wilson's Disease, and heamochromatosis.

Furthermore, there are included liver diseases caused by alcohol (e.g. ASH), non-alcoholic fatty liver changes (such as NAFLD including NASH), nutrition-mediated liver injury (for example starvation), other toxic liver injury (such as unspecific hepatitis induced by e.g. drugs such as but not limited to acetaminophen (paracetamol), chlorinated hydrocarbons (e.g. CCl₄), amiodarone (cordarone), valproate, tetracycline (only i.v.), isoniacid (Drug-induced liver disease 2004. Lazerow SK, et. al., Curr Opin Gastroenterol., 2005, 21 (3): 283-292), or food intoxication resulting in acute or chronic liver failure, e.g. by consumption of mushrooms containing aflatoxins (preferably B1 aflatoxin) or ingestion of certain metal (such as copper or cadmium) or herbal products used in natural medicine (homeopatics such as Milk thistle, Chaparral, Kawa-Kawa), interference of bilirubin metabolism, hepatitis like syndromes, cholestasis, granulomatous lesions, intrahepatic vascular lesions and cirrhosis), trauma and surgery (e.g. Pringle maneuver), radiation-mediated liver injury (such as caused by radiotherapy).

Liver disease is further understood to comprise inflammatory liver disease [caused e.g. by hepatitis B virus (HBV) and hepatitis C virus (HCV) infections] and autoimmune-mediated liver disease (e.g. autoimmune hepatitis). Further included is liver injury due to e.g. sepsis.

The term "liver disease" further comprises genetic liver disorders (such as heamochromatosis and alpha1 antitrypsin deficiency), and other inherited metabolic liver diseases [e.g. metabolic steatohepatitis (MSH)].

Pancreas diseases include all kinds of diseases that preferably affect the anatomy, physiology, metabolic, and/or genetic activities of the pancreas and hyperproliferative diseases of the pancreas. The term "hyperproliferative disease of pancreas" includes any disease related to disturbed cellular growth in pancreas. Examples of such diseases represent neoplastic diseases of pancreas, such as tumors (preferably cancer of pancreas), e.g. adenocarcinoma of pancreas, pancreas adenoma, pancreatic islet cell adenoma and pancreatic islet cell carcinoma. Furthermore, under the term of "pancreas diseases" there are included non neoplastic diseases of pancreas such as inflammatory diseases of pancreas (e.g. acute and chronic pancreatitis, and abscess), non inflammatory diseases of pancreas (e.g. metabolic disorders of pancreasr related to blood supply and coagulation), or adverse reactions in context of pancreatic transplantations, such as graft pancreatitis and pancreatic trombosis (Rosai J., Rosai and Ackerman's Surgical Pathology, 9th edition, Volume one, Mosby, Chapter 15, pg: 1061-1115).

Preferred examples of liver diseases to be treated by compounds according to the invention include liver cancers, liver cirrhosis, acute and chronic hepatitis, alcoholic liver disease (ALD), non-alcoholic fatty liver disease (NAFLD), steatosis, cholestasis, heamochromatosis and alpha1 antitrypsin deficiency.

Even more preferred embodiment for the use of compounds of formula 1 is the treatment of hyperproliferative diseases of the liver such as liver cancer, in particular hepatocarcinoma (HCC), focal nodular hyperplasis (FNH), viral and non-viral hepatitis and liver cirrhosis.

Another preferred embodiment is the treatment of hyperproliferative disorders of the pancreas, in particular pancreatic adenocarcinoma, pancreas adenoma, pancreatic islet cell adenoma and pancreatic islet cell carcinoma by using the compounds according to the invention.

Within the meaning of the invention the term "disease according to invention" encompasses diseases of liver and the pancreas as defined above.

Preferred is the use of compounds of formula (1) wherein
R¹ is optionally substituted alkyl, optionally substituted cycloalkyl, optionally substituted heterocyclyl, optionally substituted phenyl, optionally substituted phenylalkyl, heteroaryl, heteroarylalkyl, optionally further substituted heteroarylamino, or optionally substituted benzoylamino;
R² is alkyl, alkenyl, alkinyl, cycloalkyl, heterocyclyl, optionally substituted phenyl, optionally substituted phenylalkyl, optionally substituted phenoxyalkyl, heteroaryl, or heteroarylalkyl;
R³ is hydrogen or forms a ring together with R¹;
R⁴ is hydrogen;
n is zero;
A is a single bond;
B is carbonyl, carbonyloxy, carbonylamino wherein nitrogen is optionally further substitueted by alkyl or is a part of a ring, thiocarbonylamino, or sulfonyl;
and wherein the residue R¹NR³CO is connected to the carbon atom bound to nitrogen of the thiazole ring;
for treatment of diseases of liver or pancreas.

In some particularly preferred embodiments,
R¹ is cycloalkyl, optionally substituted by alkinyl or annulated by benzo, optionally substituted pyrrolidinyl, optionally substituted phenyl, optionally substituted phenylalkyl,optionally substituted thienyl, optionally substituted pyrazolyl, optionally substituted thiazolyl, optionally substituted thiadiazolyl, optionally substituted pyridyl, optionally substituted quinolinyl, optionally substituted benzofuranyl, benzopyrazinyl-alkyl-amino, optionally substituted benzoylamino, or optionally substituted thienylcarbonylamino;
R² is alkinyl, cycloalkyl, optionally substituted phenyl, optionally substituted phenylalkyl, optionally substituted phenoxyalkyl, optionally substituted pyrazolyl, optionally substituted thiazolyl, optionally substituted pyridyl, optionally substituted benzofuranyl, optionally substituted indolyl, or indolylalkyl;
R³ is hydrogen or, together with R¹, forms a piperazine ring substituted on nitrogen by optionally substituted phenyl, optionally substituted phenylalkyl, optionally substituted phenylalkenyl, optionally substituted pyridyl or pyrimidinyl;
R⁴ is hydrogen;
n is zero;
A is a single bond;
B is carbonyl, carbonyloxy, carbonylamino wherein nitrogen is optionally a part of a pyridazine ring substituted on nitrogen by pyridyl, thiocarbonylamino, or sulfonyl;
and wherein the residue R¹NR³CO is connected to the carbon atom bound to nitrogen of the thiazole ring;
for treatment of diseases of liver or pancreas.
In one particularly preferred embodiment
R¹ is 1-acetylenylcyclohexyl, 1-indanyl, N-benzyl-3-pyrrolidinyl, phenyl optionally substituted by alkyl, halo, alkoxycarbonyl, amidocarbonyl, ethylenedioxy, or oxazolyl, phenylalkyl optionally substituted by alkyl, alkoxy, halo, alkylsulfonyl or nitro, alkyl-aminocarbonylthienyl, pyrazolyl optionally substituted by alkyl and cyclopropyl, phenyl, furanyl or thienyl, thiazolyl optionally substituted by alkyl, haloalkyl-thiadiazolyl, alkoxy-pyridyl, quinolinyl optionally substituted by alkyl, alkanoyl-benzofuranyl,oxo-dihydrobenzofuranyl, benzopyrazinyl-alkyl-amino, alkoxy-benzoylamino, or alkylthienylcarbonylamino;
R² is propargyl, cyclohexyl, phenyl optionally substituted by alkyl, haloalkyl, halogen, alkoxy or dialkylamino, halo-phenylalkyl, dialkylphenoxyalkyl, phenyl-pyrazolyl, dialkyl-thiazolyl, allylmercapto-pyridyl, benzofuranyl, or indolylalkyl;
R³ is hydrogen or, together with R¹, forms a piperazine ring substituted on nitrogen by alkoxyphenyl, dihalophenyl,alkoxyphenylalkyl, phenylalkenyl, dihalo-pyridyl or pyrimidinyl;
R⁴ is hydrogen;
n is zero;
A is a single bond;
B is carbonyl, carbonyloxy, carbonylamino wherein nitrogen is optionally part of a pyridazine ring substituted on nitrogen by pyridyl, thiocarbonylamino, or sulfonyl;
and wherein the residue R¹NR³CO is connected to the carbon atom bound to nitrogen of the thiazole ring;
for treatment of diseases of liver or pancreas.

In some particularly preferred embodiments
R¹ is 1-acetylenylcyclohexyl, 1-indanyl, N-benzyl-3-pyrrolidinyl, 4-fluoro-3-nitrophenyl, 4-methyl-3-fluorophenyl, 3,4-difluorophenyl, 4-methyl-2-fluorophenyl, 4-propoxycarbonylphenyl, 2-methoxy-5-aminocarbonylphenyl, 3,4-ethylenedioxyphenyl, 3-oxazolylphenyl, 3,4-dimethoxybenzyl, 2-(4-fluorophenyl)-1,1-dimethylethyl, 4-methylsulfonylbenzyl, 2-methyl-4-chlorobenzyl, 2-(3-methoxyphenyl)ethyl, 2-nitrobenzyl, 4-methoxybenzyl, 4-methyl-3-aminocarbonyl-2-thienyl, 5-cyclopropyl-3-pyrazolyl , 5-tert. butyl-3-pyrazolyl, 5-phenyl-3-pyrazolyl, 1(2)-methyl-5-phenyl-3-pyrazolyl, 5-(2-thienyl)-3-pyrazolyl, 1 (2)-methyl-5-(2-thienyl)-3-pyrazolyl, 5-(2-furanyl)-3-pyrazolyl, 5-methyl-4-phenyl-3-pyrazolyl, 2-thiazolyl, 4-methyl-2-thiazolyl, 4,5-dimethyl-2-diazolyl, trifluoromethyl-thiadiazolyl, 6-methoxy-3-pyridyl, 6-quinolinyl, 2-methyl-6-quinolyl, 2-acetyl-6-benzofuranyl, 1-oxo-1,3-dihydro-6-benzo[c]furanyl, benzopyrazinyl-methyl-amino, 3-ethoxy-benzoylamino, or 5-methyl-2-thienylcarbonylamino;
R² is propargyl, cyclohexyl, 4-methyl-3-chlorophenyl, 4-dimethylaminophenyl, 2-trifluoromethylphenyl, 2,3,4-trimethoxyphenyl, 2,fluoro-5-chlorophenyl, 2-chlorobenzyl, 3,4-dimethylphenoxymethyl,5-phenyl-3-pyrazolyl, 4,5-dimethyl-thiazolyl, 2-allylmercapto-3-pyridyl, 2-benzofuranyl, or 3-indolylmethyl;
R³ is hydrogen or, together with R¹, forms a piperazine ring substituted on nitrogen by 2-ethoxyphenyl, 3,4-dichlorophenyl, 4-methoxybenzyl, 2-phenylvinyl, 3,5-dichloro-4-pyridyl or 2-pyrimidinyl;
R⁴ is hydrogen;
n is zero;
A is a single bond;
B is carbonyl, carbonyloxy, carbonylamino wherein nitrogen is optionally part of a pyridazine ring substituted on nitrogen by 2-pyridyl, thiocarbonylamino, or sulfonyl;
and wherein the residue R¹NR³CO is connected to the carbon atom bound to nitrogen of the thiazole ring;
for treatment of diseases of liver or pancreas. 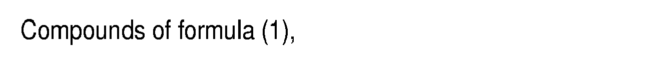 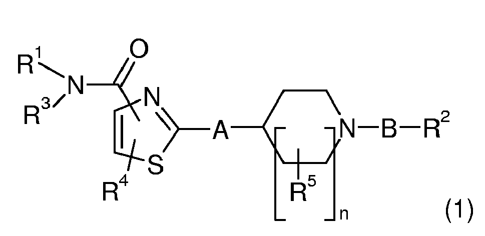 wherein
R¹ is 1-acetylenylcyclohexyl, 1-indanyl, N-benzyl-3-pyrrolidinyl, 4-fluoro-3-nitrophenyl, 4-methyl-3-fluorophenyl, 3,4-difluorophenyl, 4-methyl-2-fluorophenyl, 4-propoxycarbonylphenyl, 2-methoxy-5-aminocarbonylphenyl, 3,4-ethylenedioxyphenyl, 3-oxazolylphenyl, 3,4-dimethoxybenzyl, 2-(4-fluorophenyl)-1,1-dimethylethyl, 4-methylsulfonylbenzyl, 2-methyl-4-chlorobenzyl, 2-(3-methoxyphenyl)ethyl, 2-nitrobenzyl, 4-methoxybenzyl, 4-methyl-3-aminocarbonyl-2-thienyl, 5-cyclopropyl-3-pyrazolyl, 5-tert-butyl-3-pyrazolyl, 5-phenyl-3-pyrazolyl, 1 (2)-methyl-5-phenyl-3-pyrazolyl, 5-(2-thienyl)-3-pyrazolyl, 1 (2)-methyl-5-(2-thienyl)-3-pyrazolyl, 5-(2-furanyl)-3-pyrazolyl, 5-methyl-4-phenyl-3-pyrazolyl, 2-thiazolyl, 4-methyl-2-thiazolyl, 4,5-dimethyl-2-diazolyl, trifluoromethyl-thiadiazolyl, 6-methoxy-3-pyridyl, 6-quinolinyl, 2-methyl-6-quinolyl, 2-acetyl-6-benzofuranyl, 1-oxo-1,3-dihydro-6-benzo[c]furanyl, benzopyrazinyl-methyl-amino, 3-ethoxy-benzoylamino, or 5-methyl-2-thienylcarbonylamino;
R² is propargyl, cyclohexyl, 4-methyl-3-chlorophenyl, 4-dimethylaminophenyl, 2-trifluoromethylphenyl, 2,3,4-trimethoxyphenyl, 2,fluoro-5-chlorophenyl, 2-chlorobenzyl, 3,4-dimethylphenoxymethyl,5-phenyl-3-pyrazolyl, 4,5-dimethyl-thiazolyl, 2-allylmercapto-3-pyridyl, 2-benzofuranyl, or 3-indolylmethyl;
R³ is hydrogen or, together with R¹, forms a piperazine ring substituted on nitrogen by 2-ethoxyphenyl, 3,4-dichlorophenyl, 4-methoxybenzyl, 2-phenylvinyl, 3,5-dichloro-4-pyridyl or 2-pyrimidinyl;
R⁴ is hydrogen;
n is zero;
A is a single bond;
B is carbonyl, carbonyloxy, carbonylamino wherein nitrogen is optionally part of a pyridazine ring substituted on nitrogen by 2-pyridyl, thiocarbonylamino, or sulfonyl;
and wherein the residue R¹NR³CO is connected to the carbon atom bound to nitrogen of the thiazole ring;
are also an object of the invention as such. These compounds are particularly suitable for use in the treatment of diseases of liver or pancreas.

In particular, the compounds I to LIX listed in Table 1 are an object of the invention:

| Compound No. | EC50/µM | Chemical Structure |
|---|---|---|
| I | 5.35 | 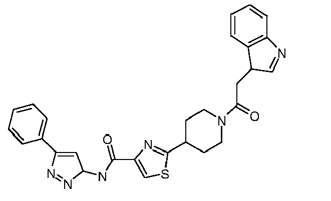 |
| II | 3.42 | 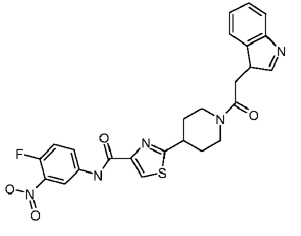 |
| III | 1.39 | 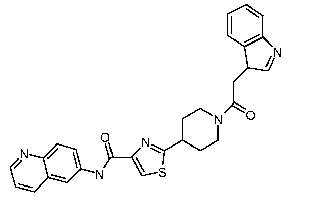 |
| IV | 3.76 | 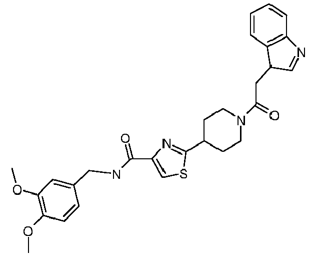 |
| V | 4.68 | 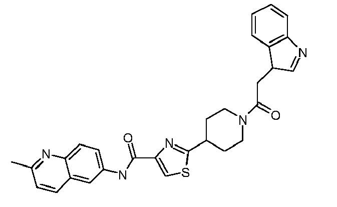 |
| VI | 4.81 | 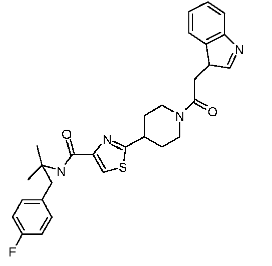 |
| VII | 3.38 | 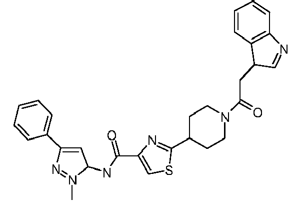 |
| VIII | 5.25 | 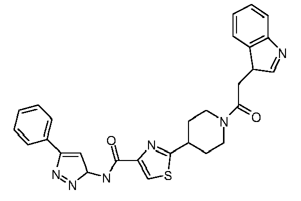 |
| IX | 12.1 | 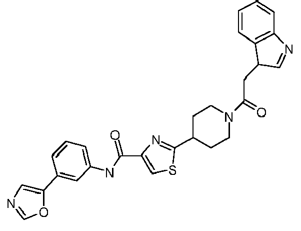 |
| X | 1.54 | 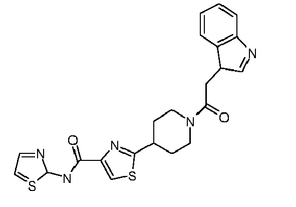 |
| XI | 0.36 | 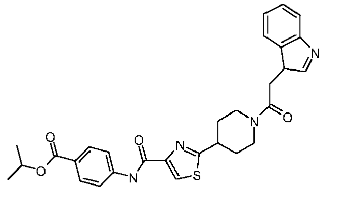 |
| XII | 13.4 | 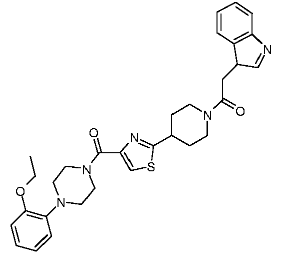 |
| XIII | 5.3 | 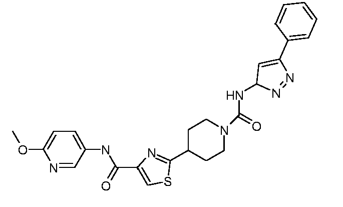 |
| XIV | 12.9 | 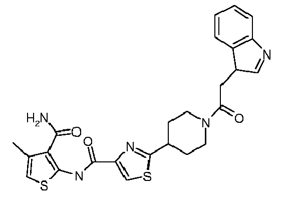 |
| XV | 1.64 | 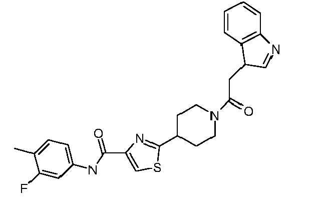 |
| XVI | 4.1 | 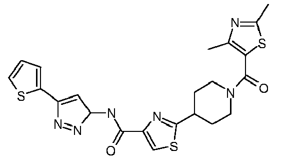 |
| XVI | 2.04 | 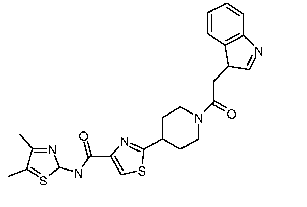 |
| XVIII | 13.4 | 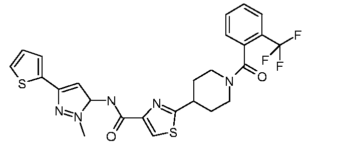 |
| XIX | 8.5 | 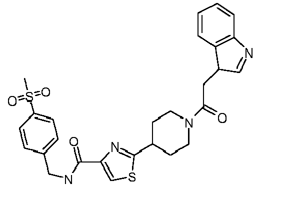 |
| XX | 5.31 | 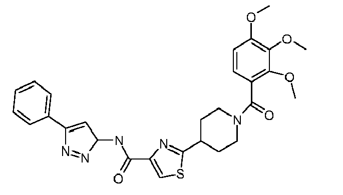 |
| XXI | 11 | 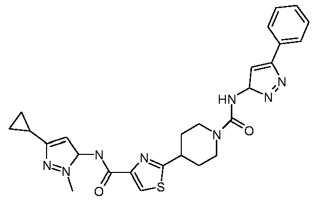 |
| XXII | 5.4 | 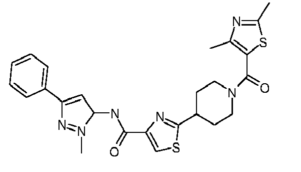 |
| XXIII | 16.7 | 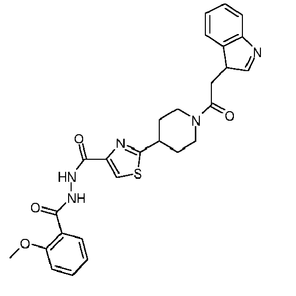 |
| XXIV | 0.25 | 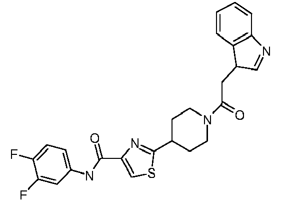 |
| XXV | 5.8 | 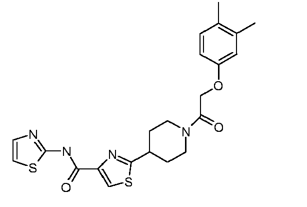 |
| XXVI | 1.15 | 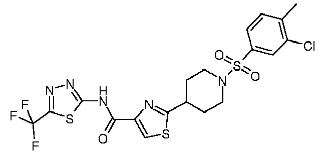 |
| XXVII | 35.6 | 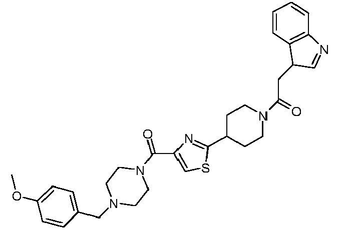 |
| XXVIII | 2.27 | 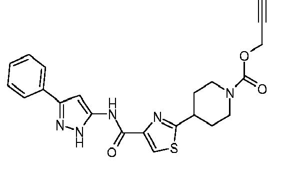 |
| XXIX | 2.7 | 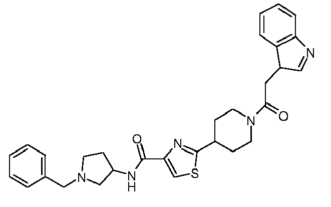 |
| XXX | 4.2 | 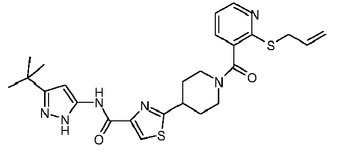 |
| XXXI | 0.5 | 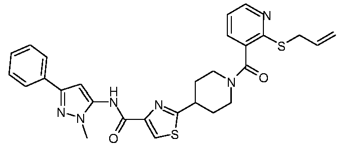 |
| XXXII | 3.08 | 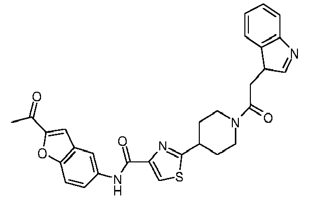 |
| XXXIII | 3.23 | 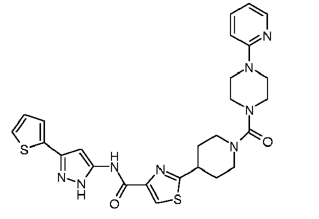 |
| XXXIV | 4.24 | 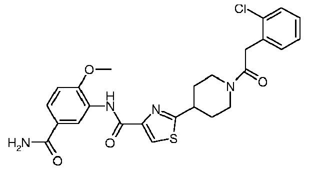 |
| XXXV | 3.5 | 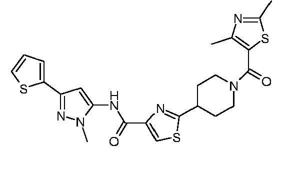 |
| XXXVI | 3.95 | 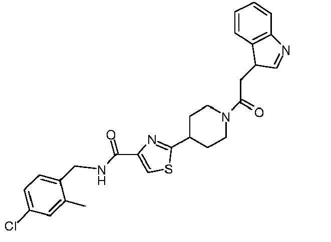 |
| XXXVII | 4.24 | 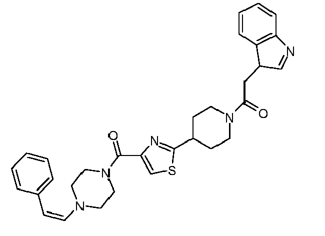 |
| XXXVIII | 20.6 | 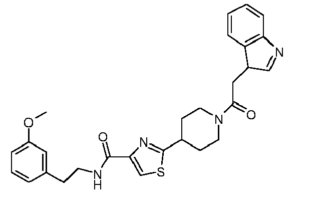 |
| XXXIX | 2.19 | 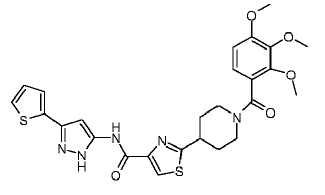 |
| XL | 2.98 | 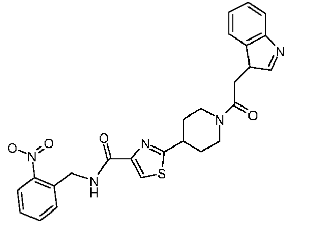 |
| XLI | 5.9 | 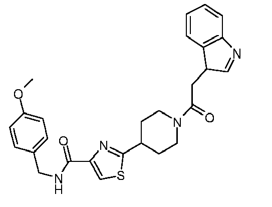 |
| XLII | 2.2 | 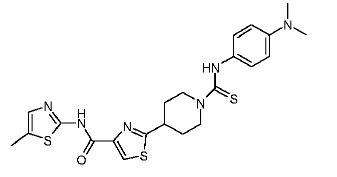 |
| XLIII | 4.74 | 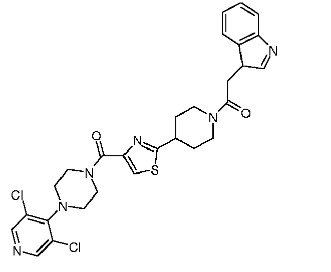 |
| XLIV | 2.53 | 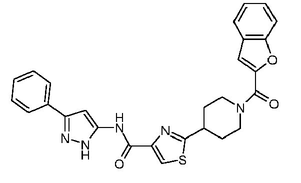 |
| XLV | 7.56 | 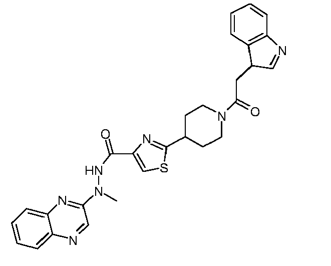 |
| XLVI | 4 | 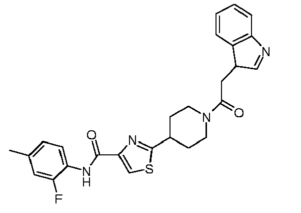 |
| XLVII | 9.15 | 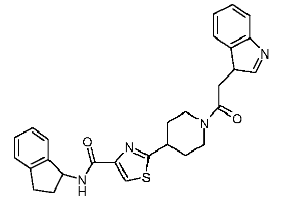 |
| XLVIII | 13.9 | 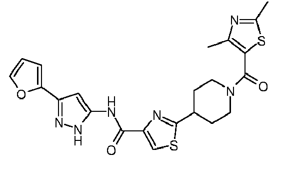 |
| XLIX | 3.03 | 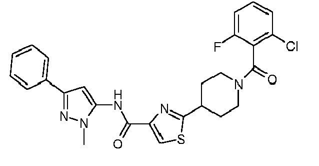 |
| L | 5.4 | 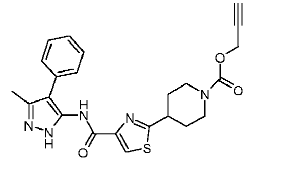 |
| LI | 4.9 | 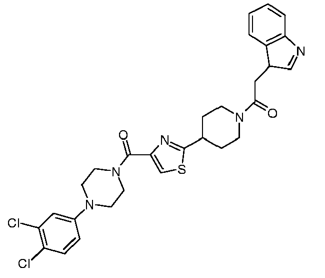 |
| LII | 19.5 | 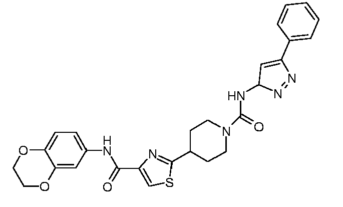 |
| LIII | 1.63 | 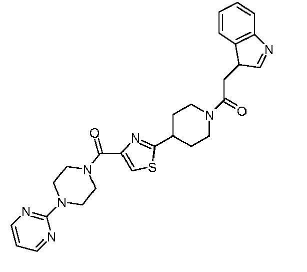 |
| LIV | 5.5 | 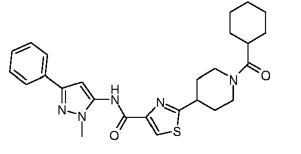 |
| LV | 4.04 | 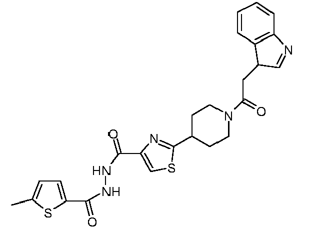 |
| LVI | 3.6 | 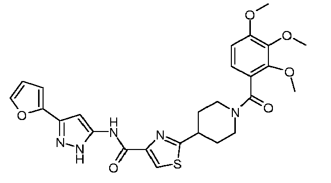 |
| LVII | 5.1 | 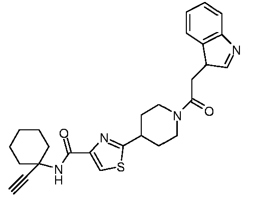 |
| LVIII | 4.58 | 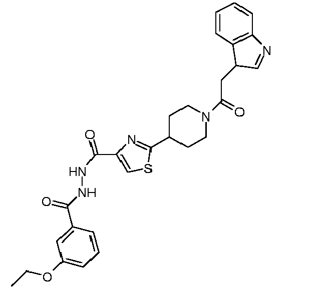 |
| LIX | 7.13 | 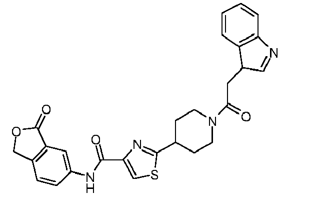 |

Particularly preferred is the compound of formula (1) wherein
R¹ is 4-isopropoxycarbonylphenyl, R² is 3-indolylmethyl, R³ is hydrogen, R⁴ is hydrogen, n is zero, A is a single bond, B is carbonyl, and wherein the residue R¹NR³CO is connected to the carbon atom bound to nitrogen of the thiazole ring.

Also particularly preferred is the compound of formula (1) wherein
R¹ is 3,4-difluorophenyl, R² is 3-indolylmethyl, R³ is hydrogen, R⁴ is hydrogen, n is zero, A is a single bond, B is carbonyl, and wherein the residue R¹NR³CO is connected to the carbon atom bound to nitrogen of the thiazole ring.

Another particularly preferred compound is the compound of formula (1) wherein
R¹ is thiazol-2-yl, R² is 3-indolylmethyl, R³ is hydrogen, R⁴ is hydrogen, n is zero, B is carbonyl, and wherein the residue R¹NR³CO is connected to the carbon atom bound to nitrogen of the thiazole ring.

Further particularly preferred is the compound of formula (1) wherein
R1 is quinolin-6-yl, R² is 3-indolylmethyl, R³ is hydrogen, R⁴ is hydrogen, n is zero, B is carbonyl, and wherein the residue R¹NR³CO is connected to the carbon atom bound to nitrogen of the thiazole ring.

Still another particularly preferred compound is the compound of formula (1) wherein
R¹ is 3-phenyl-1-methyl-1H-pyrazol-3-yl, R² is 2-[allylthio]-pyridin-3-yl, R³ is hydrogen, R⁴ is hydrogen, n is zero, B is carbonyl, and wherein the residue R¹NR³CO is connected to the carbon atom bound to nitrogen of the thiazole ring.

These particularly preferred embodiments refer to compounds no.: XI, XXIV; X; III and XXXI in Table 1.

### Method of preparation

The thiazole-piperidine compounds according to the invention, e.g. derivatives of piperidinyl-thiazole carboxylic acid and/or piperidinyl thiazole carboxamide, may be synthesized according to any of the known processes for making those compounds described in e.g. WO 2004/058750 and WO 2004/058751.

In particular, a compound of the invention may be prepared by processes that, though not applied hitherto for the new compounds of the present invention, are known *per se,* in particular by the condensation of a thiazolecarboxylic acid of formula (2) 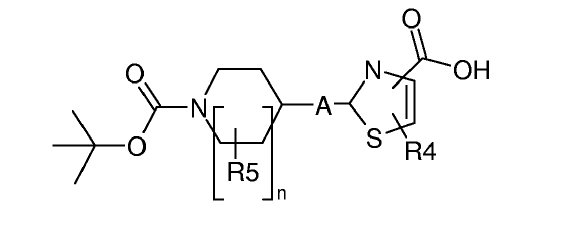 wherein the nitrogen atom in the piperidine ring may optionally be protected by a protection group equivalent to the tert-butoxycarbonyl group of formula (2), and wherein the substituents R⁴, R⁵ and A have the meanings defined under formula (1), with an amine of formula (3),

R¹-NH-R³ (3)

wherein the substituents R¹ and R³ have the meanings defined under formula (1), the protective group, e.g. tert-butoxycarbonyl, cleaved from the obtained product and the free piperidine condensed with a compound of formula (4),

HO-B-R² (4)

wherein the substituents R² and B have the meanings defined under formula (1), optionally deprotecting protected functional groups and optionally further processing substituents R¹ and R² to the desired structure. The starting compounds of formula (2), (3) and (4) are known or can be synthesized in analogy to known processes. Reaction conditions for the condensation are well known in the art, e.g. as used in peptide synthesis.

The protection of functional groups by protecting groups, the protecting groups themselves, and their removal reactions are described, for example, in standard reference books on protective groups such as T.W. Greene and P.G.M. Wuts, "Protective Groups in Organic Synthesis", Wiley, 3rd edition 1999.

All process steps described here can be carried out under known reaction conditions, in the absence of or usually in the presence of solvents or diluents, preferably such as are inert to the reagents used and able to dissolve these, in the absence or presence of catalysts, condensing agents or neutralising agents, depending on the type of reaction and/or reactants at reduced, normal, or elevated temperature, for example in the range from-100 °C to about 190°C, preferably from about -80 °C to about 150 °C, for example at -80 °C to +60 °C, at -20 °C to +40 °C, at room temperature, or at the boiling point of the solvent used, under atmospheric pressure or in a closed vessel, where appropriate under pressure, and/or in an inert atmosphere, for example under argon or nitrogen.

### Pharmaceutical preparations, methods, and uses

The present invention relates also to pharmaceutical compositions that comprise a compound of formula (1) as active ingredient and that can be used especially in the treatment of the diseases mentioned at the beginning. Compositions for enteral administration, such as nasal, buccal, rectal or, especially, oral administration, and for parenteral administration, such as intravenous, intramuscular or subcutaneous administration, to warm-blooded animals, especially humans, are especially preferred. The compositions comprise the active ingredient alone or, preferably, together with a pharmaceutically acceptable carrier or a diluent, such as, for example, physiological saline solution, demineralized water, stabilizers and/or proteinase inhibitors. The dosage of the active ingredient depends upon the disease to be treated and upon the species, its age, weight, and individual condition, the individual pharmacokinetic data, and the mode of administration.

The term "pharmaceutically acceptable" as used herein pertains to compounds, ingredients, materials, compositions, dosage, forms etc., which are within the scope of sound medical judgment, suitable for use in contact with the tissues of the subject in question (preferably human) without excessive toxicity, irritation, allergic response, or other problem or complication, commensurate with a reasonable benefit/risk ratio. Each carrier, diluent, excipient etc. must also be "acceptable" in the sense of being compatible with the other ingredients of the formulation.

The present invention relates especially to pharmaceutical compositions that comprise a compound of formula (1), a tautomer, a prodrug or a pharmaceutically acceptable salt, or a hydrate or solvate thereof, and at least one pharmaceutically acceptable carrier.

The invention relates also to pharmaceutical compositions for use in a method for the prophylactic or especially therapeutic management of the human or animal body, in particular in a method of treating of diseases of liver and/or pancreas, especially those mentioned hereinabove.

The invention relates also to processes and to the use of compounds of formula (1) thereof for the preparation of pharmaceutical preparations which comprise compounds of formula (1) as active component (active ingredient).

A pharmaceutical composition for the prophylactic or especially therapeutic management of a disease of liver and/or pancreas, of a warm-blooded animal, especially a human or a commercially useful mammal requiring such treatment, comprising a novel compound of formula (1) as active ingredient in a quantity that is prophylactically or especially therapeutically active against the said diseases, is likewise preferred.

The pharmaceutical compositions comprise from approximately 1% to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories, or capsules. Further dosage forms are, for example, ointments, creams, pastes, foams, tinctures, lip-sticks, drops, sprays, dispersions, etc. Examples are capsules containing from about 0.05 g to about 1.0 g active ingredient.

The pharmaceutical compositions of the present invention are prepared in a manner known *per se,* for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredient alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known *per se,* for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80^{®} (polyoxyethylene(20)sorbitan mono-oleate).

Suspensions in oil comprise as the oil component the vegetable, synthetic, or semisynthetic oils customary for injection purposes. In respect of such, special mention may be made of liquid fatty acid esters that contain as the acid component a long-chained fatty acid having from 8 to 22, especially from 12 to 22, carbon atoms. The alcohol component of these fatty acid esters has a maximum of 6 carbon atoms and is a monovalent or polyvalent, for example a mono-, di- or trivalent, alcohol, especially glycol and glycerol. As mixtures of fatty acid esters, vegetable oils such as cottonseed oil, almond oil, olive oil, castor oil, sesame oil, soybean oil and groundnut oil are especially useful.

The manufacture of injectable preparations is usually carried out under sterile conditions, as is the filling, for example, into ampoules or vials, and the sealing of the containers.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

Pharmaceutical compositions for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or microbicides, such as sorbic acid or benzoic acid.

In still a further aspect, the invention provides a method of therapy or prophylaxis of a patient suffering from liver disease and/or disease of pancreas which comprises the step of administering to said patient a thiazole-piperidine derivative as defined above.

The term "treatment" within the meaning of the invention refers to a treatment that preferably cures the patient from at least one disease according to the invention and/or that improves the pathological condition of the patient with respect to one or more symptoms associated with the disease, on a transient, short-term (in the order of hours to days), long-term (in the order of weeks, months or years) or permanent basis, wherein the improvement of the pathological condition may be constant, increasing, decreasing, continuously changing or oscillatory in magnitude as long as the overall effect is a significant improvement of the symptoms compared with a control patient.

Further, the term "treatment" as used herein in the context of treating liver diseases and/or diseases of the pancreas pertains generally to treatment and therapy of a human or an animal (e.g., in veterinary applications), in which some desired therapeutic effect is achieved, for example the inhibition of the progress of the condition, and includes a reduction in the rate of progress, a halt in the rate of progress, amelioration of the condition, and cure of the condition.

The term "treatment" according the invention includes combination treatments and therapies, in which two or more treatments or therapies are combined, for example sequentially or simultaneously. Treatment as a prophylactic measure (i.e. prophylaxis) is also included.

Treatment according to the invention can be carried out in a conventional manner generally known to the person skilled in the art, e.g. by means of oral application or via intravenous injection or via chemoembolization of the pharmaceutical compositions according to the invention.

Therapeutic efficacy and toxicity, e.g. ED₅₀ and LD₅₀, may be determined by standard pharmacological procedures in cell cultures or experimental animals. The dose ratio between therapeutic and toxic effects is the therapeutic index and may be expressed by the ratio LD₅₀/ED₅₀. Pharmaceutical compositions that exhibit large therapeutic indexes are preferred. The dose must be adjusted to the age, weight and condition of the individual patient to be treated, as well as the route of administration, dosage form and regimen, and the result desired, and the exact dosage should of course be determined by the practitioner.

The actual dosage depends on the nature and severity of the disease being treated, and is within the discretion of the physician, and may be varied by titration of the dosage to the particular circumstances of this invention to produce the desired therapeutic effect. In general, a suitable dose of the active compound according to invention is in the range of about 0.1 mg to about 250 mg per kilogram body weight of the subject to be treated per day. However, it is presently contemplated, that pharmaceutical compositions comprising of from about 0.1 to 500 mg/kg of the active ingredient per individual dose, preferably of from about 0.1 to 100 mg/kg, most preferred from about 0.1 to 10 mg/kg, are suitable for therapeutic treatments.

The active ingredient may be administered in one or several dosages per day. A satisfactory result can, in certain instances, be obtained at a dosage as low as 0.1 mg/kg intravenously (i.v.) and 1 mg/kg perorally (p.o.). Preferred ranges are from 0.1 mg/kg/day to about 10 mg/kg /day i.v. and from 1 mg/kg/day to about 100 mg/kg/day p.o.

Furthermore the invention relates to the manufacture of medicaments containing the compounds according to invention useful in the treatment and/or prevention of liver and/or pancreatic diseases, using standard procedures known in the prior art of mixing or dissolving the active compound with suitable pharmaceutical carriers. Such methods include the step of bringing into association the active compound with a carrier which comprises one or more accessory ingredients. In general the formulations according to invention are prepared by uniformly and intimately bringing into association the active compound with carriers (e.g. liquid carriers, finely divided solid carrier) and then shaping the product, if necessary. Suitable carriers, diluents and excipients used in the present invention can be found in standard pharmaceutical texts (see for example Handbook for Pharmaceutical Additives, 2001, 2nd edition, eds. M. Ash and I. Ash).

It will be apparent to those skilled in the art that various modifications can be made to the compositions, methods and processes of this invention. Thus, it is intended that the present invention cover such modifications and variations, provided they come within the scope of the appended claims and their equivalents. All publications cited herein are incorporated in their entireties by reference.

To practically assess the impact of the thiazole-piperidine derivatives, e.g. derivatives of piperidinyl-thiazole carboxylic acid and/or piperidinyl thiazole carboxamide, in the treatment and/or prevention of diseases according to the invention, the inhibition of cell proliferation is determined by using a luminescent cell viability assay (CellTiter-Glo^{®}; Promega, Madison, USA). The assay is based on the relation that the ATP content is directly proportional to the number of cells present in the culture. The CellTiter-Glo^{®} assay is performed to measure the proliferation rate of human hepatoma (HuH7, PLC/PRF/5, Hep3B, SK-Hep1), human hepatoblastoma (HepG2), human colon carcinoma (CX-2), human breast cancer (SK-Br-3), human ovarian carcinoma (HeLa) and human pancreatic carcinoma (DAN-G) cell lines. Compounds according to the invention exhibit EC50 values for the inhibition of HuH7 in a range of 0.36 to 35.6 µM (see Table 1). In particular, the compound according to formula (1) wherein R¹ is 4-isopropoxycarbonylphenyl, R² is 3-indolylmethyl, R³ is hydrogen, R⁴ is hydrogen, n is zero, A is a single bond, B is carbonyl, and wherein the residue R¹NR³CO is connected to the carbon atom bound to nitrogen of the thiazole ring (compound no. XI in Table 1) inhibits proliferation of the human cell lines HepG2 and Hep3B with 10.8 and 3.5 µM, respectively.

In addition compounds of the formula (1) show also inhibitory activity against tumor cell lines of the pancreas, colon, ovarian, and breast (see Table 2 in Example 2). Typically the EC50 values for inhibition of proliferation of DAN-G and HuH7 are of similar magnitude, indicating a preferred specificity for liver and pancreatic tumors. Furthermore, to assess in vivo activity of potential anti-proliferative drug candidates' standard in vivo models of liver tumors, e.g. the ectopic (subcutaneous) and orthotopic transplantation of human tumor cells in immune deficient mice like BALB/c nude or SCID mice can be used. Tumor type specificity is generated by the selection of cell lines for transplantation. In general the subcutaneously transplantation of human tumor cell lines is a general model to assess the in vivo activity of potential anti-prolerative drug candidates. Further, orthotopic transplantation models offer the ability to analyse the tumor in its typical micro environment (Armengol, C. et al., Clin. Can. Res. 2004, 10: 2150-2157).

For the subcutaneous transplantation (ectopic transplantation) of human liver tumor cells (i.e. HuH7, PLC/PRF/5, Hep3B, SK-Hep1 and HepG2) 1 to 5 million cells are subcutaneously inoculated in to the left flank of male BALC/nude mice (Yeo, E-J., et al., J. Natl. Cancer Inst. 2003, 95: 516-525). Tumor formation can be supported by inoculation of a tumor cells suspension in extra cellular matrix proteins like for example matrigel (10 mg/ml; BD biosciences). Typically tumor formation could be observed after two to four weeks, dependent on the type of tumor cells and support medium used. Treatment start could be either (1) a few days (typically 2-5) after tumor cell inoculation, or (2) after a certain tumor size (250 to 450 mm³) has been reached. The time point of treatment start allows distinguishing between inhibition of tumor formation (1) and reduction of tumor mass (2). Statistical analysis comparing tumor growth curves, tumor mass and tumor volume of serum versus placebo treated groups are finally performed.

Transplantation of human liver tumor cells into the mouse liver of immune deficient mice or mouse liver tumor cells in immune competent male mouse strains allow not only to compare tumor growth characteristics in treated and non-treated animals but also to compare survival time.

Further the DEN (N-nitroso-di-ethylamin) induced growth of liver tumors, supported by the administration of phenobarbital in C3H/He mice could be used to test the activity of potential drug candidates against e.g. liver tumors (Kemp, C. et al., Proc. Natl. Acad. Sci. USA 1989, 86: 7505-7509 and Shiota, G. et al., Carcinogenesis 1999, 20: 59-63). Male C3H/He mice are intoxicated with a single dose of DEN at age 6-8 weeks. Feeding of phenobarbital starts one week after DEN intoxication and is continued for 40 weeks. Huge enlargement of the liver is palpable even earlier. Typically the liver is enlarged around 5 to 6 times compared to non-treated mice, thus liver tumors can be macroscopically and microscopically detectable.

Treatment and/or prophylaxis of human patients with diseases according to the invention with these thiazole-piperidine compounds significantly reduce liver and/or pancreas pathology and thereby provide therapeutic and/or prophylactic efficacy as a treatment for these diseases.

When compared to the state of the art of therapy or prophylaxis of diseases of liver and pancreas the method of treatment according to the invention surprisingly provides an improved, sustained and more effective treatment.

The invention will be further illustrated below with the aid of the examples, representing preferred embodiments and features of the invention without the invention being restricted hereto.

It will be apparent to those skilled in the art that various modifications can be made to the compositions and processes of this invention. Thus, it is intended that the present invention cover such modifications and variations, provided they come within the scope of the appended claims and their equivalents. All publications cited herein are incorporated in their entireties by reference.

### Examples

*Abbreviations: eq: Equivalent(s); OMF: Dimethylformamide; RT: Room temperature; TEA: Triethylamine; DCM: Dichloromethane; TFA: Trifluoroacidic acid; MeCN: Acetonitrile; DIPEA: Diisopropylethylamine; HBTU: 2-(1H-benzotriazole-1-yl)-1,1,3,3-tetramethyluronium hexafluorophosphate; TLC: Thin layer chromatography*

### Example 1: 2-[1-{indol-3-yl-methylcarboxyl}piperidine-4-yl]-thiazole-4-yl-caboxylic acid (4-isoproxycarboxy-phenyl) amide (compound XI)

This compound corresponds to the compound according to claim 1 of formula (1) wherein R¹ is 4-isopropoxycarbonylphenyl, R² is 3-indolylmethyl, R³ is hydrogen, R⁴ is hydrogen, n is zero, A is a single bond, B is carbonyl, and wherein the residue R¹NR³CO is connected to the carbon atom bound to nitrogen of the thiazole ring.

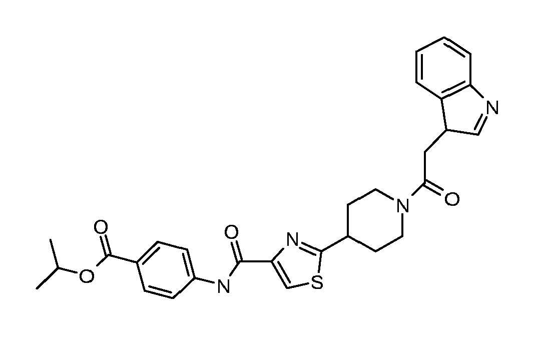

### Formation of intermediate of formula (7)

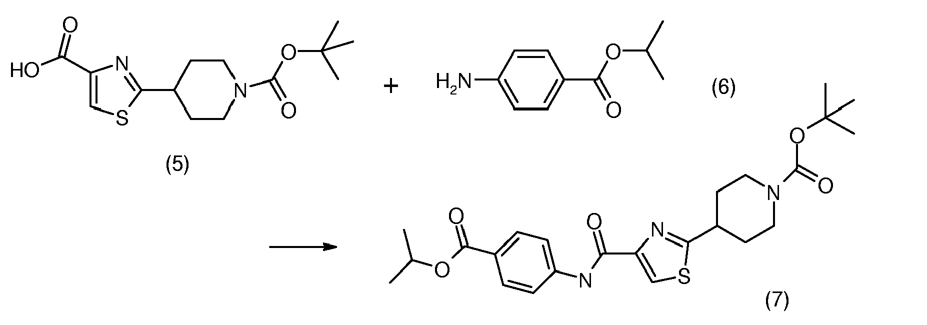

To a suspension of 2-[1-(tert-butoxycarbonyl)-4-piperidinyl]-1,3-thiazole-4-carboxylic acid (5) (1 eq.) (obtainable from Key Organics Ltd., 10R-14209), isopropyl 4-aminobenzoate (6) (0.95 eq) and HBTU (1 eq.) in acetonitile (22 ml/g) under nitrogen at room temperature DIPEA (3 eq.) is added dropwise over 30 min. Once the addition is finished, the reaction mixture is stirred over night at room temperature. TLC (ether/petroleum ether = 7:3) suggested a small amount of aniline in reaction mixture. The solvent is concentrated to obtain an oily residue. It is dissolved in ether, washed with sat. sodium carbonate solution, water and brine, dried over MgSO₄, and the solution concentrated to an oily residue. The crude product is dissolved in petroleum ether and a small (minimum) amount of ether to form a clear solution. This solution is filtered through thin silica to remove base line inpurities.
The solvent is removed from the filtrate, and the oily residue is triturated with petroleum ether to form a heavy suspension, filtered, washed well with petroleum ether, collected and dried to afford the desired product of formula (7) in 80% yield.

### Deprotection of intermediate (7)

The process is carried out under an atmosphere of nitrogen. To a solution of tert-butoxycarbonyl protected piperidine (1 eq.) of formula (7) in DCM (10 vol. eq.) at 0°C TFA (10 vol eq.) is added dropwise. The solution is left at 0°C for 10 minutes. The mixture is allowed to warm to room temperature and stirred over night. The reaction mixture is poured in to a 2 M sodium carbonate solution, and ice is added to keep the temperature at about 10°C. The organic phase is separated, and the basic aqueous solution extracted 3 times with DCM. Organic layers are combined, washed with water and brine, dried over MgSO₄, and concentrated to form a pale cream solid. The compound of formula (8) is collected and dried, yield 98%.

### Introduction of R²

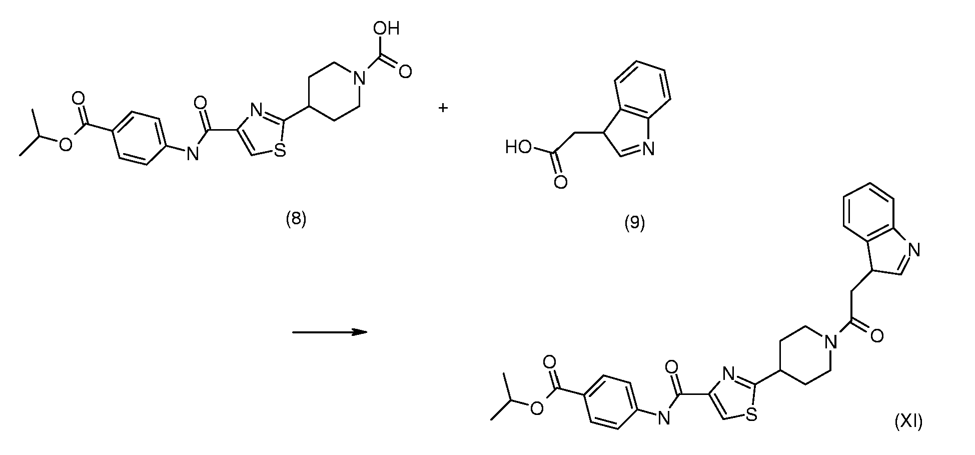

To a suspension of compound (8) (1 eq.), 1 H-indole-3-acetic acid (9) (0.95 eq.) and HBTU (1 eq.) in acetonitile (22 ml/g) under nitrogen at room temperature DIPEA (3 eq.) is added dropwise over 30 min. Once the addition is finished, the reaction mixture is stirred over night at room temperature. TLC (ethyl acetate) suggests the reaction is completed. The solvent is removed to obtain an oily residue. It is dissolved in ethyl acetate, and then washed with sat. sodium carbonate solution, water and brine, dried over MgSO₄, and concentrated to an oily residue. The crude product is purified using column chromatopgraphy, eluted with ether. Fractions containing pure product are combined, concentrated to obtain a white solid, which is collected and dried to afford the desired product, compound XI, in 60% yield.

### Example 2: Proliferation assay.

To determine the inhibition of cell proliferation a luminescent cell viability assay (CellTiter-Glo^{®}; Promega, Madison, USA) is implemented. The assay is based on the relation that the ATP content is directly proportional to the number of cells present in the culture (Technical Bulletin No. 228 "CellTiter-Glo® Luminescent Cell viability Assay", Revised 2/04. Promeg, Madison, USA). The CellTiter-Glo^{®} assay is performed to measure the proliferation rate of human hepatoma (HuH7, PLC/PRF/5, Hep3B, SK-Hep1), human hepatoblastoma (HepG2), human colon carcinoma (CX-2), human breast cancer (SK-Br-3), human ovarian carcinoma (HeLa) and human pancreatic carcinoma (DAN-G) cell lines. 3000 viable cells (viability ≥ 90%) are seeded in opaque-walled 96-well plates and incubated for 24 hours in DMEM, supplemented with 2 mM glutamin. Cell proliferation is stimulated by addition of FCS to a final concentration of 10% (v/v). Simultaneously the compounds (see Table 1 and 2) are added in a concentration range of 0 to 20 µM. Triplicates are measured for each concentration. Cells and compounds (see Table 1 and 2) are incubated for a period of 48 hours in a volume of 140 µl cell culture medium (10% (v/v) FCS, 2 mM Glutamin, DMEM). The compounds according to the invention are dissolved in 60% to 100% (v/v) DMSO at a concentration of 2 mM. The final DMSO concentration under assay conditions never exceeds 1% (v/v). Throughout the assay cells are kept in a humidified incubator (95% rH) at 37°C and 5% CO₂.

The final cell number is determined after 48 hour of incubation. Therefore 140 µl CellTiter-Glo^{®}Reagent is added in each well. The contents are mixed on an orbital shaker for 2 minutes at room temperature. After subsequent incubation for additional 10 minutes at room temperature the luminescence signal is recorded within 30 min. EC50 values are calculated using the four-parametric Hill equation (Sigma Plot).
Control values are:
1) 10% (v/v) FCS, 2 mM glutamin, DMEM -> maximum cell number
2) 2 mM glutamin, DMEM -> initial cell number

The compounds of formula (1) exhibit EC50 values in the inhibition of HuH7 in a range of 0.36 to 35.6 µM (see Table 1). In particular, the compound no. XI (see Table 1) inhibits proliferation of the human cell lines HepG2 and Hep3B with 10.8 and 3.5 µM, respectively.

In addition compounds of the formula (1) show also inhibitory activity against tumor cell lines of the pancreas, colon, ovarian, and breast (see Table 2). Typically the EC50 values for inhibition of proliferation of DAN-G and HuH7 are of similar magnitude, indicating a preferred specificity for liver and pancreatic tumors. EC50 values for DAN-G cell proliferation of compounds of formula 1 range from 1.99 to 18.3 µM (see Table 2). For most of the compounds according to the invention the EC50 value is above the highest concentration used in the proliferation assay (20 µM). However compounds of formula (1) show at least an EC50 of 9.3 µM for the inhibition of CX-2 proliferation (see Table 2). For HeLa cell the EC50 of compounds of formula 1 varies between 3.95 and 26.3 µM. Compound of the formula (1) also block proliferation of Sk-Br-3 cells (see Table 2). The observed range lies within 4.7 and 23.8 µM or higher.

**Table 2: Inhibition of DAN-G, SK-Br-3, HeLa, and CX-2 proliferation by compounds of the invention. (EC50: effective concentration reducing cell proliferation by 50%; >> much greater than).**

| Cell line | DAN-G | CX-2 | Hela | Sk-Br-3 |
|---|---|---|---|---|
| Compound No. | EC50/µM | EC50/µM | EC50/µM | EC50/µM |
| II | 3.16 | >>20 | 12.1 | 10.4 |
| III | 1.99 | 9.3 | 3.95 | 7.07 |
| IV | 7.52 | >>20 | 10,3 | >>20 |
| V | 6.9 | >>20 | 8.79 | 10.84 |
| VI | 11.2 | 19.2 | 13.5 | 10.4 |
| VII | 7.1 | >>20 | 12.5 | 10.8 |
| VIII | 7.65 | 10.1 | 9.33 | 4.7 |
| X | 2.53 | >>20 | 17.99 | 18.9 |
| XI | 7.63 | 22.3 | 26.3 | 10.2 |
| XIII | 8.9 | >>20 | 10.05 | 12.3 |
| XV | 3.81 | 10.9 | 17.2 | 10.8 |
| XVI | 7.99 | >>20 | 10.5 | >>20 |
| XVII | 5.59 | >>20 | 23.5 | 18.9 |
| XX | 7.8 | >>20 | 10.33 | 19.7 |
| XXIV | 2.55 | 10.3 | 8.91 | 16.6 |
| XXVI | 13.5 | 20.2 | 11.1 | 19.3 |
| XXXI | 3.58 | 10.4 | 20.5 | 10.2 |
| XXXIII | 13.84 | >>20 | 11.6 | >>20 |
| XL | 9.01 | 14.9 | 22.9 | 23.8 |
| XLVII | 9.7 | 19.9 | 13.6 | 11.2 |
| LIII | 18.2 | >>20 | 23.6 | 19.5 |
| LVI | 16.95 | >>20 | 10.5 | >>20 |

## Claims

1. Use of a compund of formula (1) 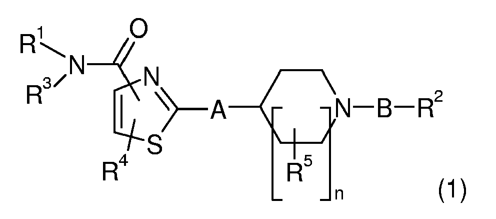 wherein
R¹ is optionally substituted alkyl, alkenyl, cycloalkyl, heterocyclyl, aryl, arylalkyl, heteroaryl, heteroarylalkyl, alkoxy, amino, alkylamino, dialkylamino, alkanoylamino, aroylamino, carboxyalkyl, alkoxycarbonyl, aryloxycarbonyl, or cyano;
R² is optionally substituted alkyl, alkenyl, alkinyl, cycloalkyl, heterocyclyl, aryl, arylalkyl, aryloxyalkyl, heteroaryl, heteroarylalkyl, or carboxyalkyl;
R³ is hydrogen or forms a ring together with R¹;
R⁴ is hydrogen or alkyl,
R⁵ is alkyl and n is any number from zero to nine;
A is a single bond or alkylene;
B is a single bond, carbonyl, carbonyloxy, carbonylmercapto, carbonylamino wherein nitrogen is optionally further substitueted by alkyl or being part of a ring, thiocarbonyl, thiocarbonyloxy, thiocarbonylmercapto, thiocarbonylamino, sulfonyl,or sulfonylamino;
and wherein the residue R¹NR³CO and R⁴ can be connected either to the carbon atom bound to sulfur or bound to nitrogen of the thiazole ring and the alkyl residues (R⁵)ₙ may be bound to any carbon atom of the piperidine ring;
for the preparation of a medicament for the treatment or prophylaxis of diseases of liver or pancreas.

2. The use according to claim 1 of a compound of formula (1) wherein
R¹ is optionally substituted alkyl, optionally substituted cycloalkyl, optionally substituted heterocyclyl, optionally substituted phenyl, optionally substituted phenylalkyl, heteroaryl, heteroarylalkyl, optionally further substituted heteroarylamino, or optionally substituted benzoylamino;
R² is alkyl, alkenyl, alkinyl, cycloalkyl, heterocyclyl, optionally substituted phenyl, optionally substituted phenylalkyl, optionally substituted phenoxyalkyl, heteroaryl, or heteroarylalkyl;
R³ is hydrogen or forms a ring together with R¹;
R⁴ is hydrogen;
n is zero;
A is a single bond;
B is carbonyl, carbonyloxy, carbonylamino wherein nitrogen is optionally further substitueted by alkyl or being part of a ring, thiocarbonylamino, or sulfonyl;
and wherein the residue R¹NR³CO is connected to the carbon atom bound to nitrogen of the thiazole ring.

3. The use according to claim 1 of a compound of formula (1) wherein
R¹ is 1-acetylenylcyclohexyl, 1-indanyl, N-benzyl-3-pyrrolidinyl, phenyl optionally substituted by alkyl, halo, alkoxycarbonyl, amidocarbonyl, ethylenedioxy, or oxazolyl, phenylalkyl optionally substituted by alkyl, alkoxy, halo, alkylsulfonyl or nitro, alkyl-aminocarbonylthienyl, pyrazolyl optionally substituted by alkyl and cyclopropyl, phenyl, furanyl or thienyl, thiazolyl optionally substituted by alkyl, haloalkyl-thiadiazolyl, alkoxy-pyridyl, quinolinyl optionally substituted by alkyl, alkanoyl-benzofuranyl,oxo-dihydrobenzofuranyl, benzopyrazinyl-alkyl-amino, alkoxy-benzoylamino, or alkylthienylcarbonylamino;
R² is propargyl, cyclohexyl, phenyl optionally substituted by alkyl, haloalkyl, halogen, alkoxy or dialkylamino, halo-phenylalkyl, dialkylphenoxyalkyl, phenyl-pyrazolyl, dialkyl-thiazolyl, allylmercapto-pyridyl, benzofuranyl, or indolylalkyl;
R³ is hydrogen or, together with R¹, forms a piperazine ring substituted on nitrogen by alkoxyphenyl, dihalophenyl, alkoxyphenylalkyl, phenylalkenyl, dihalo-pyridyl or pyrimidinyl;
R⁴ is hydrogen;
n is zero;
A is a single bond;
B is carbonyl, carbonyloxy, carbonylamino wherein nitrogen is optionally being part of a pyridazine ring substituted on nitrogen by pyridyl, thiocarbonylamino, or sulfonyl;
and wherein the residue R¹NR³CO is connected to the carbon atom bound to nitrogen of the thiazole ring.

4. A compound of formula (1), 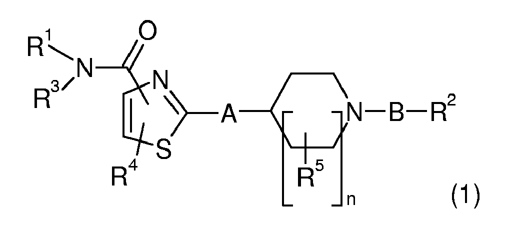 wherein
R¹ is 1-acetylenylcyclohexyl, 1-indanyl, N-benzyl-3-pyrrolidinyl, 4-fluoro-3-nitrophenyl, 4-methyl-3-fluorophenyl, 3,4-difluorophenyl, 4-methyl-2-fluorophenyl, 4-propoxycarbonylphenyl, 2-methoxy-5-aminocarbonylphenyl, 3,4-ethylenedioxyphenyl, 3-oxazolylphenyl, 3,4-dimethoxybenzyl, 2-(4-fluorophenyl)-1,1-dimethylethyl, 4-methylsulfonylbenzyl, 2-methyl-4-chlorobenzyl, 2-(3-methoxyphenyl)ethyl, 2-nitrobenzyl, 4-methoxybenzyl, 4-methyl-3-aminocarbonyl-2-thienyl, 5-cyclopropyl-3-pyrazolyl , 5-tert. butyl-3-pyrazolyl, 5-phenyl-3-pyrazolyl, 1 (2)-methyl-5-phenyl-3-pyrazolyl, 5-(2-thienyl)-3-pyrazolyl, 1 (2)-methyl-5-(2-thienyl)-3-pyrazolyl, 5-(2-furanyl)-3-pyrazolyl, 5-methyl-4-phenyl-3-pyrazolyl, 2-thiazolyl, 4-methyl-2-thiazolyl, 4,5-dimethyl-2-diazolyl, trifluoromethyl-thiadiazolyl, 6-methoxy-3-pyridyl, 6-quinolinyl, 2-methyl-6-quinolyl, 2-acetyl-6-benzofuranyl, 1-oxo-1,3-dihydro-6-benzo[c]furanyl, benzopyrazinyl-methyl-amino, 3-ethoxy-benzoylamino, or 5-methyl-2-thienylcarbonylamino;
R² is propargyl, cyclohexyl, 4-methyl-3-chlorophenyl, 4-dimethylaminophenyl, 2-trifluoromethylphenyl, 2,3,4-trimethoxyphenyl, 2,fluoro-5-chlorophenyl, 2-chlorobenzyl, 3,4-dimethylphenoxymethyl,5-phenyl-3-pyrazolyl, 4,5-dimethyl-thiazolyl, 2-allylmercapto-3-pyridyl, 2-benzofuranyl, or 3-indolylmethyl;
R³ is hydrogen or, together with R¹, forms a piperazine ring substituted on nitrogen by 2-ethoxyphenyl, 3,4-dichlorophenyl, 4-methoxybenzyl, 2-phenylvinyl, 3,5-dichloro-4-pyridyl or 2-pyrimidinyl;
R⁴ is hydrogen;
n is zero;
A is a single bond;
B is carbonyl, carbonyloxy, carbonylamino wherein nitrogen is optionally being part of a pyridazine ring substituted on nitrogen by 2-pyridyl, thiocarbonylamino, or sulfonyl;
and wherein the residue R¹NR³CO is connected to the carbon atom bound to nitrogen of the thiazole ring.

5. A compound selected from the group consisting of
the compound according to claim 4 of formula (1) wherein
R¹ is 4-isopropoxycarbonylphenyl, R² is 3-indolylmethyl, R³ is hydrogen, R⁴ is hydrogen, n is zero, A is a single bond, B is carbonyl, and wherein the residue R¹NR³CO is connected to the carbon atom bound to nitrogen of the thiazole ring;
the compound according to claim 4 of formula (1) wherein
R¹ is 3,4-difluorophenyl, R² is 3-indolylmethyl, R³ is hydrogen, R⁴ is hydrogen, n is zero, A is a single bond, B is carbonyl, and wherein the residue R¹NR³CO is connected to the carbon atom bound to nitrogen of the thiazole ring:
the compound according to claim 4 of formula (1), wherein
R¹ is thiazol-2-yl, R² is 3-indolylmethyl, R³ is hydrogen, R⁴ is hydrogen, n is zero, B is carbonyl, and wherein the residue R¹NR³CO is connected to the carbon atom bound to nitrogen of the thiazole ring;
the compound according to claim 4 of formula (1), wherein
R1 is quinolin-6-yl, R² is 3-indolylmethyl, R³ is hydrogen, R⁴ is hydrogen, n is zero, B is carbonyl, and wherein the residue R¹NR³CO is connected to the carbon atom bound to nitrogen of the thiazole ring; and
the compound according to claim 4 of formula (1), wherein
R¹ is 3-phenyl-1-methyl-1 H-pyrazol-5-yl, R² is 2-[allylthio]-pyridin-3-yl, R³ is hydrogen, R⁴ is hydrogen, n is zero, B is carbonyl, and wherein the residue R¹NR³CO is connected to the carbon atom bound to nitrogen of the thiazole ring.

6. A pharmaceutical composition comprising a compound of claim 4.

7. The use according to any of claim 1 to 3, wherein the liver disease is a disease selected from the group consisting of liver cancer, the intrahepatic cholangioma, benign liver neoplasms and focal nodular hyperplasia, viral and non-viral hepatitis, liver cirrhosis, alcoholic liver disease, non-alcoholic fatty liver disease, steatosis, cholestasis, nutrition-mediated liver injury, toxic liver injury, infectious liver disease, liver injury in sepsis, autoimmune-mediated liver disease, hemochromatosis, alpha1 antitrypsin deficiency, radiation-mediated liver injury.

8. The use according to claim 7, wherein the liver disease is a liver cancer.

9. The use according to claim 8, wherein the liver cancer is hepatocarcinoma.

10. The use according to any of claims 1 to 3, wherein the disease of pancreas is a disease selected from the group consisting of neoplastic diseases and non neoplastic diseases of pancreas.

11. The use according to claim 10, wherein the neoplastic disease of pancreas is a cancer of pancreas.

12. Use of a compound according to claim 4 or 5 for treatment or prophylaxis of diseases of liver or pancreas according to one of claims 7 to 11.

13. A method of treating patients with diseases of liver or pancreas which comprises administering to the patient in need thereof a therapeutically effective amount of a compound according to one of claims 1 to 5.
